(19) Europäisches Patentamt
European Patent Office
Office européen des brevets

(11) EP 3 138 491 A1

(12) EUROPEAN PATENT APPLICATION

(43) Date of publication:
08.03.2017 Bulletin 2017/10

(51) Int Cl.:
A61B 5/145 (2006.01)    A61B 5/1486 (2006.01)

(21) Application number: 16186294.1

(22) Date of filing: 30.08.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 01.09.2015  JP 2015172308

(71) Applicants:
• SYSMEX CORPORATION
Kobe-shi
Hyogo 651-0073 (JP)
• Mie University
Mie 514-8507 (JP)

(72) Inventors:
• Suminaka, Chihiro
Hyogo, 651-0073 (JP)
• Sato, Toshiyuki
Hyogo, 651-0073 (JP)
• Yano, Yutaka
Mie, 514-8507 (JP)
• Suzuki, Toshinari
Mie, 514-8507 (JP)
• Uemura, Mei
Mie, 514-8507 (JP)

(74) Representative: Hoffmann Eitle
Patent- und Rechtsanwälte PartmbB
Arabellastraße 30
81925 München (DE)

(54) METHOD AND APPARATUS FOR ANALYZING CONCENTRATION STATE OF MEASUREMENT TARGET COMPONENT IN BLOOD

(57) Disclosed is a method for analyzing a concentration state of a measurement target component in blood, the method including: obtaining a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid; obtaining a value of an area under a blood concentration time curve of the measurement target component on the basis of the value regarding the amount of the measurement target component; and calculating a value regarding a ratio of a time period during which a concentration of the measurement target component has exceeded a predetermined concentration relative to the extraction time period, on the basis of the value of the area under the blood concentration time curve.

FIG. 3

**Description**

FIELD OF THE INVENTION

The present invention relates to a method and an apparatus for analyzing the concentration state of a measurement target component in blood.

BACKGROUND

From the viewpoint of keeping QOL in a diabetic patient and selecting an appropriate therapy, management of the blood glucose state is indispensable. A hyperglycemic state is harmful for tissues in the body and grasping the presence/absence of the hyperglycemic state is very important for diabetes therapies. In recent years, continuous glucose monitoring called CGM is more and more used. By use of CGM, it is possible to grasp the state of blood glucose level of higher or lower than a certain level. However, CGM requires a sensor to be subcutaneously inserted into a patient, thus causing a large burden on the patient.

Thus, International Publication WO 2010/013808 describes obtaining, without subcutaneously inserting a sensor into a subject, the value of the area under the blood concentration-time curve, i.e., the value of AUC, of a measurement target component such as glucose contained in tissue fluid extracted for a predetermined time period from the skin of the subject.

SUMMARY OF THE INVENTION

The scope of the present invention is defined solely by the appended claims, and is not affected to any degree by the statements within this summary.

The technique described in International Publication WO 2010/013808 allows obtaining the AUC value in a predetermined time period, but does not address how to grasp, by using the AUC value, whether the measurement target component is in a high concentration state, how long the high concentration state has continued, or the like.

A method for analyzing a concentration state of a measurement target component in blood according to an aspect includes: obtaining a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid; obtaining a value of an area under a blood concentration time curve of the measurement target component on the basis of the value regarding the amount of the measurement target component; and calculating a value regarding a ratio of a time period during which a concentration of the measurement target component has exceeded a predetermined concentration relative to the extraction time period of the measurement target component, on the basis of the value of the area under the blood concentration time curve.

A method for analyzing a concentration state of a measurement target component in blood according to another aspect includes: obtaining a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid; obtaining a value of an area under a blood concentration time curve of the measurement target component on the basis of the value regarding the amount of the measurement target component; comparing the value of the area under the blood concentration time curve with a predetermined threshold value; and determining, on the basis of a result of the comparison, whether a concentration of the measurement target component has exceeded a predetermined concentration.

An apparatus configured to analyze a concentration state of a measurement target component in blood according to an aspect includes: a controller configured to: obtain a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid; obtain a value of an area under a blood concentration time curve of the measurement target component on the basis of the value regarding the amount of the measurement target component; and calculate a value regarding a ratio of a time period during which a concentration of the measurement target component has exceeded a predetermined concentration relative to the extraction time period of the measurement target component, on the basis of the obtained value of the area under the blood concentration time curve.

An apparatus configured to analyze a concentration state of a measurement target component in blood according to another aspect includes: a controller configured to: obtain a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid; obtain a value of an area under a blood concentration time curve of the measurement target component on the basis of the obtained value regarding the amount of the measurement target component; and compare the obtained value of the area under the blood concentration time curve with a predetermined threshold value, and determine whether a concentration of the measurement target component has exceeded a predetermined concentration on the basis of a result of the comparison.

BRIEF DESCRIPTION OF THE DRAWINGS

FIG. 1 is a block diagram showing a concentration state analyzer for a measurement target component in blood according to an embodiment;
FIG. 2 is a graph for explaining an AUC value;
FIG. 3 is a flow chart for describing an analysis process performed by the concentration state analyzer;
FIG. 4 is a perspective view showing one example of a blood glucose AUC value measurement apparatus, a sensor chip, and a collection member;
FIG. 5 is a schematic plan view showing the blood glucose AUC value measurement apparatus shown in FIG. 2;
FIG. 6 is a schematic side view showing the blood glucose AUC value measurement apparatus shown in FIG. 2;
FIG. 7 is a schematic cross-sectional view showing the collection member to be used in measurement of the blood glucose AUC;
FIG. 8 is a perspective view showing a puncture tool to be used in measurement of the blood glucose AUC;
FIG. 9 is a diagram for describing a measurement procedure of a blood glucose AUC measurement method;
FIG. 10 is a diagram for describing the measurement procedure of the blood glucose AUC measurement method;
FIG. 11 is a diagram for describing the measurement procedure of the blood glucose AUC measurement method;
FIG. 12 is a schematic side view showing another example of the blood glucose AUC value measurement apparatus;
FIG. 13 is a graph showing a result of examination of Example 1;
FIG. 14 is a graph showing a result of examination of Example 2;
FIG. 15 is a graph showing a result of examination of Example 3;
FIG. 16 is a graph showing a result of examination of Example 4; and
FIG. 17 is a graph showing a result of examination of Example 5.

DETAILED DESCRIPTION OF THE PREFERRED EMBODIMENTS

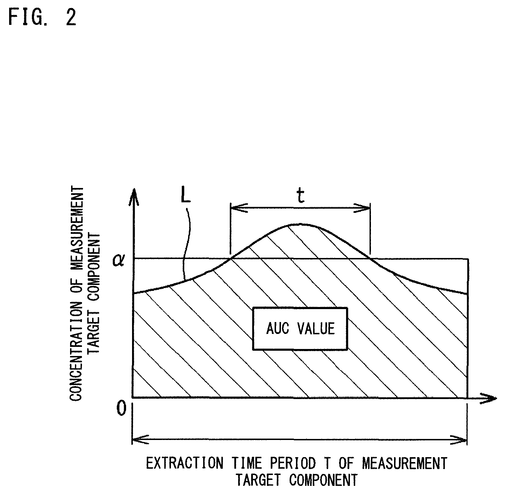

As a value that reflects the total amount of a component that has circulated in an organism within a predetermined period, an area under the blood concentration time curve (AUC) is known. As shown by hatching in FIG. 2, the AUC means the area surrounded by a curve (*in vivo* component blood concentration-time curve) L being a graph representing the blood concentration of a predetermined *in vivo* component over lapse of time, and by the horizontal axis (time axis).
A blood glucose AUC is the area (unit: mg·h/dl) of the portion surrounded by a curve being a graph representing the blood glucose level over lapse of time, and by the horizontal axis. The blood glucose AUC can be an index to be used in effect determination of an oral preparation in diabetes therapies. For example, if a value reflecting the total amount of glucose (blood glucose) that has circulated in blood within a predetermined period after a glucose load had been applied (after meal) is measured by use of the blood glucose AUC, it is possible to estimate the total amount of glucose that has circulated in the body of the subject after the glucose load had been applied.
An analysis method and an analyzer according to embodiments described below further analyze a high concentration state of a measurement target component such as glucose, from the AUC value. Specifically, the analysis method and the analyzer analyze the ratio of a time period during which the concentration of a measurement target component has exceeded a predetermined concentration, relative to the extraction time period of the measurement target component. Further, the analysis method and the analyzer also analyze whether the concentration of the measurement target component has exceeded a predetermined concentration. Through these analyses, a high concentration state of the measurement target component can be easily grasped, and thus can be utilized in diagnosis and therapies of diabetes and the like.

[Summary of embodiments]

A method for analyzing a concentration state of a measurement target component in blood according to an embodiment includes: obtaining a value of an area under a blood concentration time curve of the measurement target component; and calculating a value regarding a ratio of a time period during which a concentration of the measurement target component has exceeded a predetermined concentration relative to the extraction time period of the measurement target component, on the basis of the value of the area under the blood concentration time curve.

An analyzer configured to analyze a concentration state of a measurement target component in blood according to an embodiment includes a controller configured to: obtain a value of an area under a blood concentration time curve of the measurement target component; and calculate a value regarding a ratio of a time period during which a concentration of the measurement target component has exceeded a predetermined concentration relative to an extraction time period of the measurement target component, on the basis of the obtained value of the area under the blood concentration time curve. In the description below, the "value of the area under the blood concentration time curve of the measurement target component" may be simply referred to as "AUC value", and the "value regarding the ratio of the time period during which the concentration of the measurement target component has exceeded a predetermined concentration relative to the extraction time period of the measurement target component" may be simply referred to as a "value regarding the ratio".

The above method and apparatus for analyzing the concentration state calculate, by using the value of the area under the blood concentration time curve of the measurement target component, a value regarding the ratio of the time period during which the concentration of the measurement target component has exceeded the predetermined concentration relative to the extraction time period of the measurement target component. Accordingly, it is possible to grasp, more easily than by means of the AUC value, how long a high concentration state of the measurement target component has continued. For example, it is possible to grasp, more easily than by means of the AUC value, how long a hyperglycemic state of the subject having received a glucose load has continued.

The value of the area under the blood concentration time curve obtained for analysis of the concentration state of a measurement target component can be obtained on the basis of: the value regarding the amount of the measurement target component contained in tissue fluid extracted from the organism for a predetermined time period; the value regarding the amount of an electrolyte; and the extraction time period of the tissue fluid. The value of the area under the blood concentration time curve in this case is an estimated value obtained from the value regarding the amount of the measurement target component and the like. Accordingly, for example, in order to measure the concentration of the measurement target component, it is no longer necessary to subcutaneously embed a sensor into the subject, or to collect blood many times in one day, which can reduce the burden on the patient. However, in order to obtain the value regarding the ratio of the time period during which the concentration of the measurement target component has exceeded the predetermined concentration relative to the extraction time period of the measurement target component, it is also possible to use the value of the area under the blood concentration time curve directly obtained from the concentration of the measurement target component collected by subcutaneously embedding a sensor into the subject, or the concentration of the measurement target component collected through a plurality of blood collections.

It is recommended that the extraction time period of the measurement target component is not less than 60 minutes. The time period of 60 minutes is a sufficient time period for grasping how long a high concentration state of the measurement target component continues. However, the extraction time period of the measurement target component may be not less than 120 minutes, or not less than 180 minutes. For example, in such a case where tissue fluid is extracted by utilizing the time period during which the subject is sleeping, the extraction time period of the measurement target component can be extended, and can be set to 8 hours, for example.

The tissue fluid can be extracted by a collection member attached to the skin of the organism. Accordingly, the tissue fluid can be easily extracted. To the organism, a process for accelerating the extraction of the tissue fluid can be performed. This process can be a process of forming fine pores in the skin of the organism. The collection member can accumulate the tissue fluid extracted through the fine pores. It is possible to use a collection member in which a support member having an adhesive layer supports an extraction medium, and the support member can be attached to the skin by means of the adhesive layer. The collection member can contain a gel as the extraction medium. In this case, due to the moisture absorbing ability of the gel, tissue fluid can be extracted from the skin of the organism. The gel is preferably a polyvinyl alcohol gel.

The gel can contain a hypertonic aqueous solution which has a higher osmotic pressure than pure water. Due to the osmotic pressure of the hypertonic aqueous solution having a higher osmotic pressure than pure water, transfer of the tissue fluid to the extraction medium is accelerated. The hypertonic aqueous solution may or may not contain an auxiliary component different from the measurement target component. The auxiliary component can be at least one member selected from the group consisting of potassium chloride, glycine, and urea. In this case, the concentration of the auxiliary component is preferably not less than 0.2 mmol/L.

The value regarding the ratio of the time period during which the concentration of the measurement target component has exceeded the predetermined concentration relative to the extraction time period of the measurement target component may be calculated by generating a regression equation from the relationship between measurement values of known concentrations of the measurement target component and the value of the area under the blood concentration time curve obtained from the measurement values, and by applying the AUC value to the regression equation.

Specifically, the value regarding the ratio of the time period during which the concentration of the measurement target component has exceeded the predetermined concentration relative to the extraction time period of the measurement target component is preferably calculated by applying the value of the area under the blood concentration time curve to the following formula.

[Formula 1]

$$y = \frac{a}{1 + b \times exp(-cx)} \quad \cdots (1)$$

where y is the value regarding the ratio of the time period during which the concentration of the measurement target component has exceeded the predetermined concentration, x is the value of the area under the blood concentration time curve, and a, b, and c are each a constant.

This formula represents a so-called sigmoid curve. The present inventors examined the relationship between the value regarding the ratio of the time period during which the concentration of the measurement target component has exceeded the predetermined concentration relative to the extraction time period of the measurement target component, and the value of the area under the blood concentration time curve. Then, the inventors have found that the transition of the change in these values can be approximated by a sigmoid curve. On the basis of the above founding, in the analysis method and analyzer according to an embodiment, the value of the area under the blood concentration time curve is put into the above formula, thereby obtaining the value regarding the ratio of the time period during which the concentration of the measurement target component has exceeded the predetermined concentration relative to the extraction time period of the measurement target component. As the constants a, b, and c, appropriate values are selected depending on the extraction time period of the measurement target component and a predetermined concentration of the measurement target component.

The measurement target component may be glucose. In this case, the predetermined concentration of glucose may be a value in the range of not less than 180 mg/dL and not greater than 200 mg/dL; 200 mg/dL; or 180 mg/dL. Alternatively, the predetermined concentration of glucose may be a value less than 180 mg/dL or a value that exceeds 200 mg/dL. The electrolyte may be sodium ion. The electrolyte is not limited to sodium ion, and may be an inorganic ion such as potassium ion, chloride ion, or the like.

A method for analyzing a concentration state of a measurement target component in blood according to another embodiment includes: obtaining a value of an area under a blood concentration time curve of the measurement target component; comparing the value of the area under the blood concentration time curve with a predetermined threshold value; and determining, on the basis of a result of the comparison, whether a concentration of the measurement target component has exceeded a predetermined concentration.

An analyzer configured to analyze a concentration state of a measurement target component in blood according to another embodiment includes a controller configured to: obtain a value of an area under a blood concentration time curve of the measurement target component; compare the obtained value of the area under the blood concentration time curve with a predetermined threshold value; and determine, on the basis of a result of the comparison, whether a concentration of the measurement target component has exceeded a predetermined concentration.

The above method and apparatus for analyzing the concentration state can determine whether the concentration of the measurement target component has exceeded the predetermined concentration, by using the value of the area under the blood concentration time curve of the measurement target component. That is, by use of the value of the area under the blood concentration time curve, the concentration state of the measurement target component can be indirectly grasped.

The analyzer can include: a set portion in which to set a collection member in which an electrolyte and a measurement target component in tissue fluid have been accumulated in advance, the tissue fluid having been extracted for a predetermined extraction time period of not less than 60 minutes from an organism which has been subjected to a process for accelerating extraction of the tissue fluid; a detection portion configured to obtain values regarding amounts of the measurement target component and the electrolyte accumulated in the collection member set in the set portion; and an analysis portion configured to obtain a value of the area under the blood concentration time curve of the measurement target component which is an integrated value that corresponds to a predetermined extraction time period not less than 60 minutes of the concentration of the measurement target component *in vivo,* on the basis of the value regarding the amount of the measurement target component and the value regarding the amount of the electrolyte.

[Details of embodiments]

Hereinafter, an analyzer which analyzes the concentration state of a measurement target component such as glucose by using the value of the area under the blood concentration time curve, i.e., the AUC value, and an AUC measurement apparatus which measures the AUC value will be described in details with reference to the drawings.

[Concentration state analyzer]

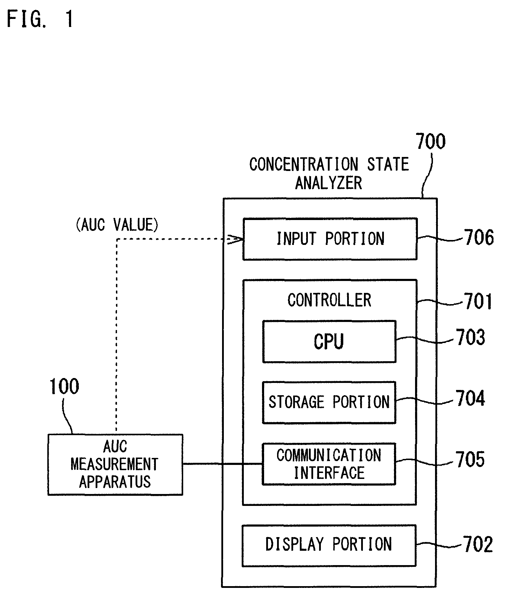

As shown in FIG. 1, a concentration state analyzer 700 is an apparatus which obtains an AUC value measured by an AUC measurement apparatus 100 and which analyzes the concentration state of the measurement target component from the obtained AUC value. The concentration state analyzer 700 includes a controller 701, a display portion 702, and an input portion 706. The controller 701 is composed of a CPU 703, a storage portion 704 such as ROM, RAM, hard disk, and the like, and a communication interface 705. By the CPU 703 executing computer programs stored in the storage portion 704, the controller 701 exhibits predetermined functions.

In the storage portion 704, set values, data values, and the like to be used in arithmetic processing performed by the CPU 703 are stored in addition to the computer programs. AUC values obtained from the AUC measurement apparatus 100 are also stored in the storage portion 704, and are read out as appropriate for arithmetic processing. The communication interface 705 is connected to the AUC measurement apparatus 100. The controller 701 can transmit/receive instruction signals and data to/from the AUC measurement apparatus 100 via the communication interface 705. Specifically, the controller 701 can receive via the communication interface 705 an AUC value measured by the AUC measurement apparatus 100.

The display portion 702 is formed by a liquid crystal monitor and the like. The display portion 702 displays an analysis result obtained through processing performed in the controller 701, as well as various types of information that a user should be notified of. The input portion 706 is composed of a keyboard, a mouse, a touch panel, and the like, and receives various types of information inputted from outside by the user. The input portion 706 can receive an input of an AUC value measured by the AUC measurement apparatus 100, for example. In this case, the AUC measurement apparatus 100 and the analyzer 700 may not be connected to each other via the communication interface 705, and the controller 701 can execute an analysis process by using the AUC value inputted from the input portion 706. The input portion 706 can receive an input for selecting either one of a first analysis process and a second analysis process described later.

The controller 701 executes the first analysis process and the second analysis process by using the AUC value measured by the AUC measurement apparatus 100. The first analysis process is a process of calculating a value regarding the ratio of the time period during which the concentration of the measurement target component has exceeded a predetermined concentration, relative to the extraction time period of the measurement target component, which is the AUC value measurement time period. The process result of the first analysis process will also be referred to as a first analysis value. The second analysis process is a process of determining whether the concentration of the measurement target component has exceeded a predetermined concentration. The process result of the second analysis process will also be referred to as a second analysis value. The controller 701 can select and execute either one of the first analysis process and the second analysis process. The user can input via the input portion 706 information regarding which of the first analysis process and the second analysis process is to be executed by the controller 701.

(First analysis process)

As shown in FIG. 2, when the horizontal axis represents time and the vertical axis represents concentration of the measurement target component, the area of hatching surrounded by the horizontal axis and a blood concentration-time curve L is the AUC value. In the first analysis process, when the extraction time period of the measurement target component is defined as T, and the time period during which the concentration of the measurement target component has exceeded a predetermined concentration $\alpha$ is defined as t, the ratio of the time period t during which the concentration of the measurement target component has exceeded the predetermined concentration $\alpha$, relative to the extraction time period T of the measurement target component, i.e., the value of t/T, is calculated as a first analysis value.

Specifically, the controller 701 puts the AUC value measured by the AUC measurement apparatus 100 into the prediction formula (1) as described above, thereby calculating the first analysis value.

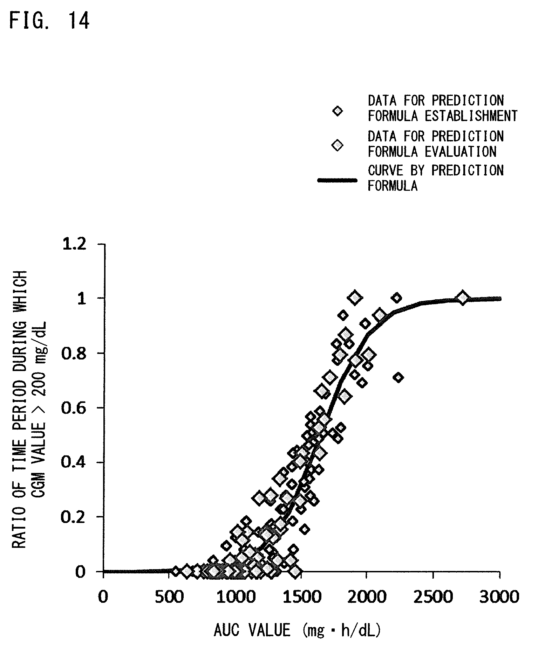

The larger the AUC value is, the larger the value regarding the ratio of the time period during which the concentration of the measurement target component has exceeded the predetermined concentration becomes. However, the value does not constantly increase in association with the increase of the AUC value of the measurement target component. In a region where the AUC value is small and in a region where the AUC value is large, the increase in the value regarding the ratio of the time period during which the concentration of the measurement target component has exceeded the predetermined concentration is mild. For example, as indicated by the "curve by prediction formula" in FIG. 14, in a region where the AUC value is small, the increase in the value regarding the ratio of the time period during which the concentration of the measurement target component has exceeded the predetermined concentration is also mild. Then, the degree of increase in the value regarding the ratio of the time period during which the concentration of the measurement target component has exceeded the predetermined concentration is increased. However, in the region where the AUC value is large, the increase becomes mild again. Therefore, the relationship between the AUC value and the value regarding the ratio of the time period during which the concentration of the measurement target component has exceeded the predetermined concentration can be approximated by a regression curve, i.e., a sigmoid curve, depicted according to the above prediction formula. By using the above prediction formula, the controller 701 obtains, from the AUC value, the value regarding the ratio of the time period during which the concentration of the measurement target component has exceeded the predetermined concentration, which is the first analysis value.

With respect to the constants a, b, and c of the prediction formula, values that correspond to the AUC value measurement time period, and values that correspond to the predetermined concentration of the measurement target component are stored in the storage portion 704 in advance. For the constants a, b, and c, values that correspond to the obtained AUC value measurement time period and the predetermined concentration of the measurement target component are read out from the storage portion 704, and used in the arithmetic processing by the CPU 703. When the measurement target component is glucose, the predetermined concentration is set in a range of not less than 180 mg/dL and not greater than 200 mg/dL.

The concentration values 180 mg/dL and 200 mg/dL are values used in general as determination references in diabetes diagnosis. For example, 200 mg/dL is used as a reference when determining diabetes on the basis of blood glucose level after two hours in a 75 g oral glucose tolerance test. 200 mg/dL is also used as a reference when determining diabetes on the basis of casual blood glucose level measured at a desired time. 180 mg/dL is used as a reference for determining diabetes on the basis of blood glucose level after one hour in an oral glucose tolerance test. 180 mg/dL is used as a reference when determining gestational diabetes on the basis of blood glucose level after one hour in a 75g oral glucose tolerance test. Further, as the blood glucose control target value for a diabetic dialysis patient, the casual blood glucose level is set to a value less than 180 mg/dL to 200 mg/dL. On the basis of the setting inputted in advance, the controller 701 executes processing, using a value in the range of not less than 180 mg/dL and not greater than 200 mg/dL. The predetermined concentration can be inputted and set by the user via the input portion 706. The predetermined concentration is not limited to a value in a range of not less than 180 mg/dL and not greater than 200 mg/dL. Either one of 180 mg/dL and 200 mg/dL may be selected and set. The predetermined concentration may be set to a value outside the range of not less than 180 mg/dL and not greater than 200 mg/dL.

(Second analysis process)

There is a certain correlation between the magnitude of the AUC value and the concentration of the measurement target component. Therefore, in the second analysis process, instead of comparing the concentration of the measurement target component with a predetermined concentration, the AUC value is compared with a predetermined threshold value, and on the basis of the comparison result, whether the concentration of the measurement target component exceeds the predetermined concentration is determined. The predetermined concentration of the measurement target component is set to either one of 200 mg/dL and 180 mg/dL as described above. The controller 701 selects either one of 200 mg/dL and 180 mg/dL on the basis of the setting inputted in advance. However, the predetermined concentration is not limited to these values, and may be changed as appropriate.

(Concentration state analysis procedure)

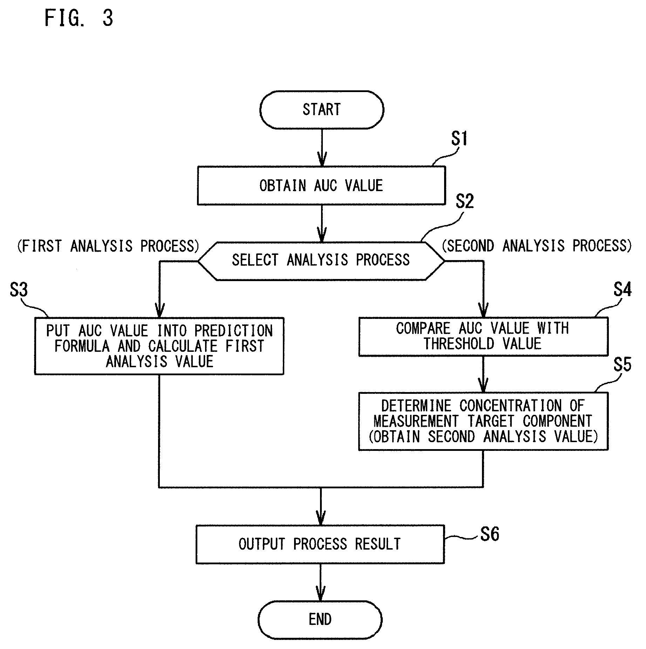

Next, the procedure of the analysis performed by the concentration state analyzer 700 will be described. As shown in FIG. 3, in step S1, the controller 701 performs a step of obtaining an AUC value measured by the AUC measurement apparatus 100. Next, in step S2, the controller 701 selects either one of the first analysis process and the second analysis process. This selection is performed on the basis of the information inputted in advance by the user via the input portion 706.

When the first analysis process has been selected, the controller 701 puts the obtained AUC value into the above prediction formula, and then, calculates a value, i.e., the first analysis value, regarding the ratio of the time period during which the concentration of the measurement target component has exceeded the predetermined concentration, relative to the extraction time period of the measurement target component. Then, in step S6, the controller 701 causes the display portion 702 to display the first analysis value.

When the second analysis process has been selected in step S2, the controller 701 compares, in step S4, the obtained AUC value with a predetermined threshold value. In step S5, on the basis of the result of the comparison between the AUC value and the predetermined threshold value, the controller 701 determines whether the concentration of the measurement target component exceeds the predetermined concentration, and obtains the second analysis value indicating the determination result. Then, in step S6, the controller 701 causes the display portion 702 to display the second analysis value.

The measurement target component concentration state analyzer 700 may be an apparatus that performs only one of the first analysis process and the second analysis process. Alternatively, the concentration state analyzer 700 may perform the first analysis process and the second analysis process in parallel, and output process results of both analysis processes. The AUC value to be used in the concentration state analyzer 700 can be measured by an AUC measurement apparatus described next as an example. The AUC measurement apparatus can analyze the concentration state of the measurement target component.

[One example of AUC measurement apparatus]

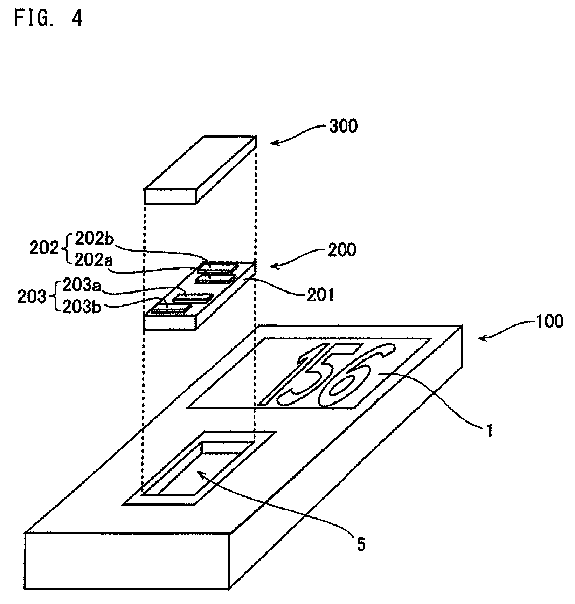

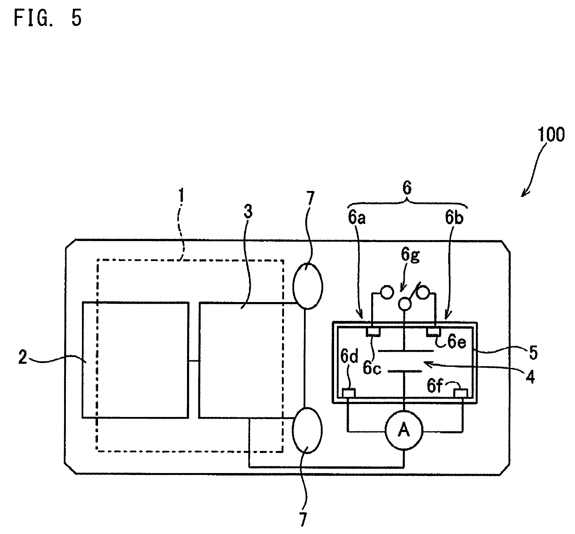

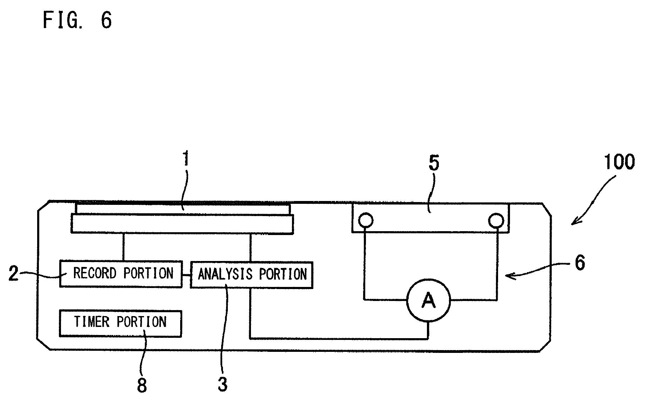

As shown in FIG. 4 to FIG. 6, a measurement apparatus 100 for measuring an AUC value includes: a display portion 1; a record portion 2; an analysis portion 3; a power source 4; a set portion 5 in which to set a sensor chip 200 and a gel 301 of a collection member 300; an electric circuit 6 to be connected to the sensor chip 200 set in the set portion 5; an operation button 7 to be used by a subject for operating the measurement apparatus 100; and a timer portion 8. This AUC measurement apparatus 100 is an apparatus that measures an AUC value on the basis of tissue fluid extracted from a patient through slight invasion by a puncture tool 400 and by use of the collection member 300 which are described later. Hereinafter, the technique of extracting tissue fluid through such slight invasion may be referred to as a minimally invasive tissue fluid extraction technique.

The display portion 1 has a function of displaying a measurement result obtained by the analysis portion 3 and data recorded in the record portion 2. The record portion 2 is provided in order to save data in the past. The analysis portion 3 has a function of calculating a glucose concentration and an electrolyte concentration (Na) on the basis of an output value from the electric circuit 6. The set portion 5 is formed in a recessed shape and can have the sensor chip 200 and the gel 301 of the collection member 300 set therein by housing them. The electric circuit 6 includes a glucose measurement circuit 6a and an electrolyte measurement circuit 6b. The glucose measurement circuit 6a includes terminals 6c and 6d exposed in the set portion 5. The electrolyte measurement circuit 6b includes terminals 6e and 6f exposed in the set portion 5. The electric circuit 6 includes a switch 6g for performing switching between the glucose measurement circuit 6a and the electrolyte measurement circuit 6b. The user can switch between the glucose measurement circuit 6a and the electrolyte measurement circuit 6b by operating the operation button 7 so as to operate the switch 6g. The operation button 7 is provided in order to perform operations such as switching of the switch 6g, switching between displays on the display portion 1, and setting the timer portion 8. In order to end extraction of glucose after a lapse of a predetermined time period after the start of the extraction, the timer portion 8 has a function of notifying the user of the extraction end time.

(Configuration of sensor chip)

As shown in FIG. 4, the sensor chip 200 includes: a plastic substrate 201; a pair of glucose measurement electrodes 202 provided on the upper surface of the substrate 201; and a pair of electrolyte measurement electrodes 203 provided on the upper surface of the substrate 201. The glucose measurement electrodes 202 consist of a working electrode 202a obtained by forming a GOD enzyme film (GOD: glucose oxidase) on a platinum electrode, and a counter electrode 202b which is a platinum electrode. The electrolyte measurement electrodes 203 consist of a working electrode 203a composed of silver/silver chloride and a counter electrode 203b composed of silver/silver chloride. In a state where the sensor chip 200 is set in the set portion 5 of the measurement apparatus 100, the working electrode 202a and the counter electrode 202b of the glucose measurement electrodes 202 come into contact with the terminals 6c and 6d of the glucose measurement circuit 6a, respectively. Similarly, in a state where the sensor chip 200 is set in the set portion 5 of the measurement apparatus 100, the working electrode 203a and the counter electrode 203b of the electrolyte measurement electrodes 203 come into contact with the terminals 6e and 6f of the electrolyte measurement circuit 6b, respectively. The electric circuit 6 described above, and the glucose measurement electrodes 202 and the electrolyte measurement electrodes 203 of the sensor chip 200 form a detection portion for obtaining values regarding the amount of the measurement target component and the amount of the electrolyte.

(Configuration of collection member)

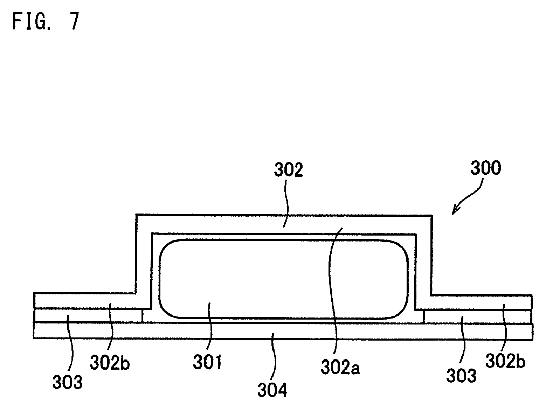

As shown in FIG. 7, the collection member 300 is used for extracting tissue fluid by the minimally invasive tissue fluid extraction technique described above. The collection member 300 has a structure in which the gel 301 is supported by a support member 302, the gel 301 having non-conductivity (containing substantially no electrolyte) and moisture absorbing ability that allows the gel 301 to hold tissue fluid having oozed out from inside the subject to the skin due to passive diffusion. The gel 301 is made of polyvinyl alcohol. The support member 302 has a recessed portion 302a and a flange portion 302b which is formed on the periphery side of the recessed portion 302a. The gel 301 is held in the recessed portion 302a. An adhesive layer 303 is provided on a surface of the flange portion 302b. In a state before measurement, a peel-off paper 304 which seals the gel 301 held in the recessed portion 302a is attached by means of the adhesive layer 303. When the measurement is performed, the peel-off paper 304 is peeled off whereby the gel 301 and the adhesive layer 303 are exposed, and the gel 301 can be fixed by being attached to the skin of the subject by means of the adhesive layer 303 in a state where the gel 301 is in contact with the skin of the subject.

The gel 301 is configured to be able to accumulate the measurement target component in the tissue fluid extracted for not less than 60 minutes from an organism. More specifically, the gel 301 has a sufficient volume that allows accumulation of the measurement target component in the tissue fluid extracted for not less than 60 minutes from an organism. Such a volume of the gel 301 is determined depending on the extraction time period of the tissue fluid. Specifically, if the extraction time period is 60 minutes, the volume of the gel is preferably not less than 33 μL. If the extraction time period is 120 minutes, not less than 66 μL is preferable. If the extraction time period is 180 minutes, not less than 100 μL is preferable.

(Configuration of puncture tool)

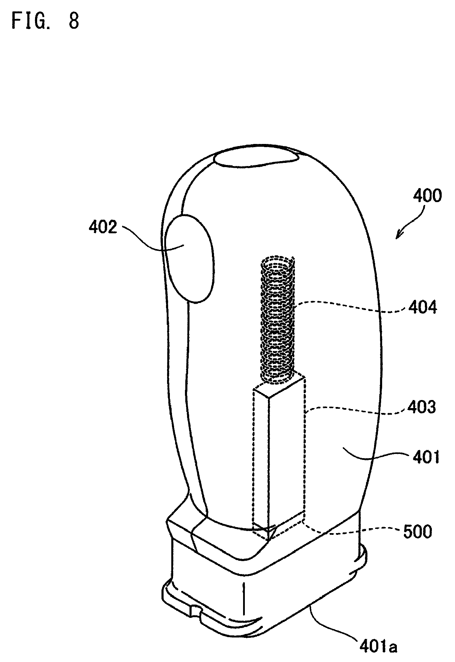

As shown in FIG. 8, the puncture tool 400 is a device which has attached thereto a microneedle tip 500 having a large number of sterilized microneedles, and which forms fine pores for extracting tissue fluid in a skin 600 of a subject by bringing the microneedles of the microneedle tip 500 into contact with the epidermis of an organism, specifically, in contact with the skin 600 of the subject. Each microneedle of the microneedle tip 500 has such a length that allows, when a fine pore is formed by the puncture tool 400, the fine pore to penetrate the epidermis of the skin but that does not allow the fine pore to reach a deep part of the corium. The puncture tool 400 includes a housing 401, a release button 402 provided on a surface of the housing 401, and an array chuck 403 and a spring member 404 which are provided inside the housing 401. An opening (not shown) is formed in a lower part 401a of the housing 401. The spring member 404 has a function of urging the array chuck 403 downward. The array chuck 403 can have the microneedle tip 500 attached to the lower end thereof. A plurality of microneedles are formed on the lower surface of the microneedle tip 500. In addition, the puncture tool 400 includes a fixing mechanism (not shown) for fixing the array chuck 403 in a state where the array chuck 403 is pushed upward against the urging force of the spring member 404. When the subject presses down the release button 402, fixation of the array chuck 403 by the fixing mechanism is released, the array chuck is projected downward by the urging force of the spring member 404, and the microneedle tip 500 collides with the skin, whereby fine pores are formed in the skin.

(Blood glucose AUC measurement method)

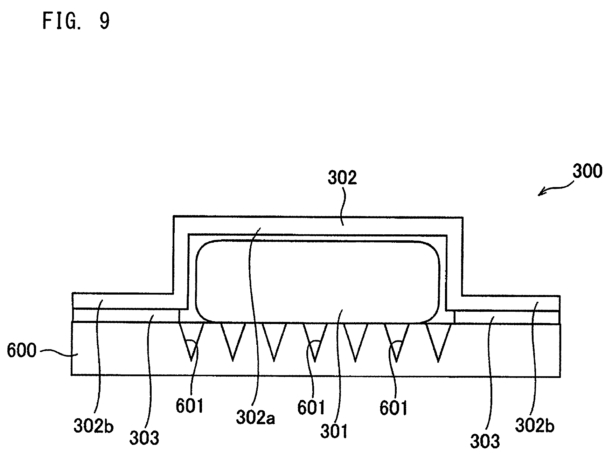

Next, with reference to FIG. 9 to FIG. 11, a measurement procedure of a blood glucose AUC measurement method according to an embodiment will be described. First, the subject cleans the skin 600 with alcohol or the like, to remove substances (sweat, dust, etc.) which could be disturbance factors for the result of the measurement. Then, after the cleaning, the subject forms fine pores in the skin by means of the puncture tool 400 having the microneedle tip 500 attached thereto.

Next, the subject operates the operation button 7 to set a time period for the timer portion 8 of the measurement apparatus 100. Any time period can be set as long as the time period is not less than 60 minutes. Here, an example in which 180 minutes is set will be described.

Next, the subject removes the peel-off paper 304 of the collection member 300 shown in FIG. 7, and attaches the collection member 300 to the skin such that the gel 301 is located at the site where fine pores 601 are formed, as shown in FIG. 9. Accordingly, the gel 301 comes into contact with the site where the fine pores 601 are formed, and tissue fluid containing glucose and the electrolyte starts to move through the fine pores 601 to the gel 301, whereby extraction is started. Moreover, the subject turns on the timer portion 8 of the measurement apparatus 100 at the same time of the start of the extract. Thereafter, the collection member 300 is left in a state of being attached to the skin 600, until a predetermined time period (the set time period of the alarm) elapses. Then, at the time when the predetermined time period has elapsed and the alarm sounds, the subject removes the collection member 300 from the skin 600. Here, since the alarm of the timer portion 8 is set to 180 minutes, extraction is continuously performed for 180 minutes. Then, the extraction-accumulation step ends.

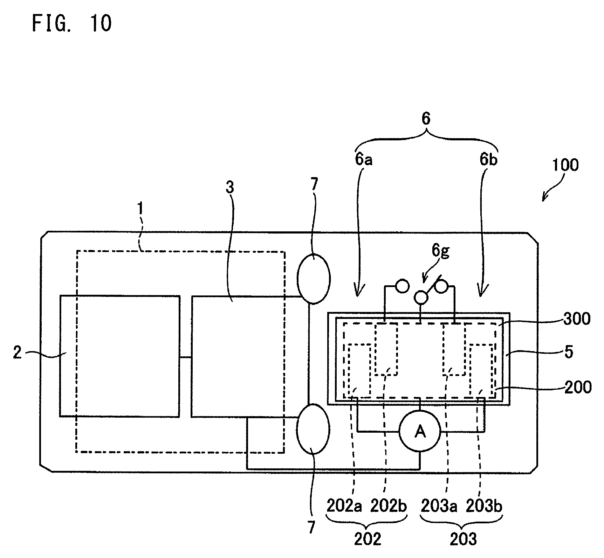

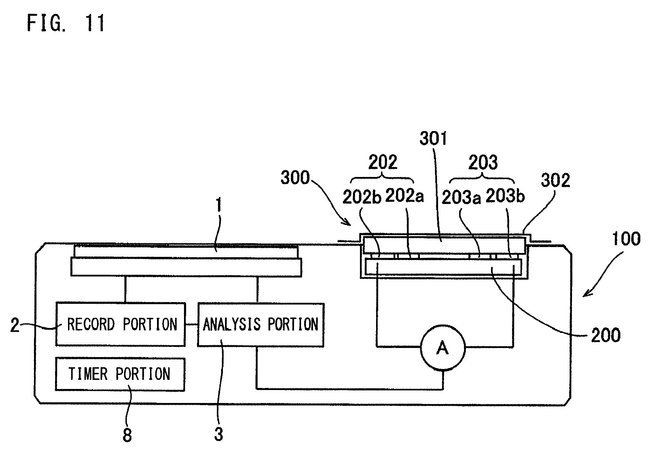

Next, as shown in FIG. 10 and FIG. 11, the subject sets the sensor chip 200 in the set portion 5 of the measurement apparatus 100, and sets the gel 301 of the collection member 300 on the sensor chip 200. Accordingly, a first circuit is formed by the glucose measurement circuit 6a of the measurement apparatus 100, the glucose measurement electrodes 202 of the sensor chip 200, and the gel 301 of the collection member 300, and a second circuit is formed by the electrolyte measurement circuit 6b of the measurement apparatus 100, the electrolyte measurement electrodes 203 of the sensor chip 200, and the gel 301 of the collection member 300.

When measuring the concentration of the extracted glucose, the subject switches the switch 6g to the glucose measurement circuit 6a by means of the operation button 7, and gives an instruction to start the measurement. Accordingly, a constant voltage is applied to the first circuit, and a current value I (glc) detected by an ammeter is inputted to the analysis portion 3. Here, the following formula (2) is established between the current value I (glc) and a glucose concentration C (glc) of the gel 301.

$$C \text{ (glc)} = A \times I \text{ (glc)} + B \text{ (A and B are each a constant)} \cdots (2)$$

The analysis portion 3 calculates the glucose concentration C (glc) from the current value I (glc) on the basis of formula (2) above.

Further, by using the obtained glucose concentration C (glc) and the amount of an extraction solvent, i.e., a volume V of the gel, the analysis portion 3 calculates an extracted glucose amount M (glc) on the basis of formula (3) below.

$$M \text{ (glc)} = C \text{ (glc)} \times V \cdots (3)$$

When measuring the concentration of the extracted electrolyte, the subject switches the switch 6g to the electrolyte measurement circuit 6b by means of the operation button 7, and gives an instruction to start the measurement. Accordingly, a constant voltage is applied to the second circuit, and a current value I (ele) detected by the ammeter is inputted to the analysis portion 3. Here, the following formula (4) is established between the current value I (ele) and an electrolyte concentration C (Na) of the gel 301.

$$C \text{ (Na)} = C \times I \text{ (ele)} + D \text{ (C and D are each a constant)} \cdots (4)$$

The analysis portion 3 calculates the electrolyte concentration C (Na) from the current value I (ele) on the basis of formula (4) above.

Further, by using the electrolyte concentration C (Na), the volume V of the gel 301, and the extraction time period t, the analysis portion 3 calculates an extraction speed J of the electrolyte at the extraction site, on the basis of formula (5) below.

$$J = C \text{ (Na)} \times V \times 1/t \cdots (5)$$

Then, from the calculated electrolyte extraction speed J, the analysis portion 3 calculates a glucose permeability P (glc) indicating the ease of extracting glucose on the basis of formula (6) below.

$$P \text{ (glc)} = E \times J + F \text{ (E and F are each a constant)} \cdots (6)$$

Formula (6) is obtained as follows. The glucose permeability P (glc) indicating the ease of extracting glucose is normally given by a ratio (this ratio will be tentatively referred to as a true glucose permeability P' (glc)) of the blood glucose AUC obtained through blood collection, relative to the amount of extracted glucose. The true glucose permeability P' (glc) shows a certain correlation with the electrolyte extraction speed J. Therefore, by obtaining an approximation formula on the basis of the electrolyte extraction speed J and the true glucose permeability P' (glc), the above formula (6) can be obtained.

According to formula (6) above, the glucose permeability P (glc) indicating the ease of extracting glucose can be obtained on the basis of the electrolyte extraction speed J that can be obtained without performing blood collection.

On the basis of formula (7) below, the analysis portion 3 calculates a predicted AUC, which is a predicted blood glucose AUC, from the extracted glucose amount M (glc) obtained by formula (3) and the glucose permeability P (glc) obtained by formula (6).

$$\text{Predicted AUC} = M \text{ (glc)} / P \text{ (glc)} \cdots (7)$$

The predicted blood glucose AUC is a value having a high correlation with a collected-blood blood glucose AUC which is calculated by performing a plurality of blood collections. The value of this predicted blood glucose AUC is displayed on the display portion 1, and recorded in the record portion 2. Then, the measurement step ends.

In the embodiment above, an exemplary configuration has been described in which, in order to measure the predicted AUC, the analysis portion 3 calculates the glucose concentration C (glc), the glucose amount M (glc), the electrolyte concentration C (Na), the electrolyte extraction speed J, and the glucose permeability P (glc). However, the above configuration may not be employed. For example, formula (7) for calculating the predicted AUC can be replaced by formula (8) on the basis of formulae (2) to (6).

$$\text{Predicted AUC} = \{(A \times I\,(glc) + B) \times t\} / [E \times (C \times I\,(ele) + D) \times F]\ (A\ to\ F\ are\ each\ a\ constant) \cdots (8)$$

Therefore, by using formula (8) above, the analysis portion 3 can directly calculate the predicted AUC on the basis of the current value I (glc) and the current value I (ele).

[Another example of AUC measurement apparatus]

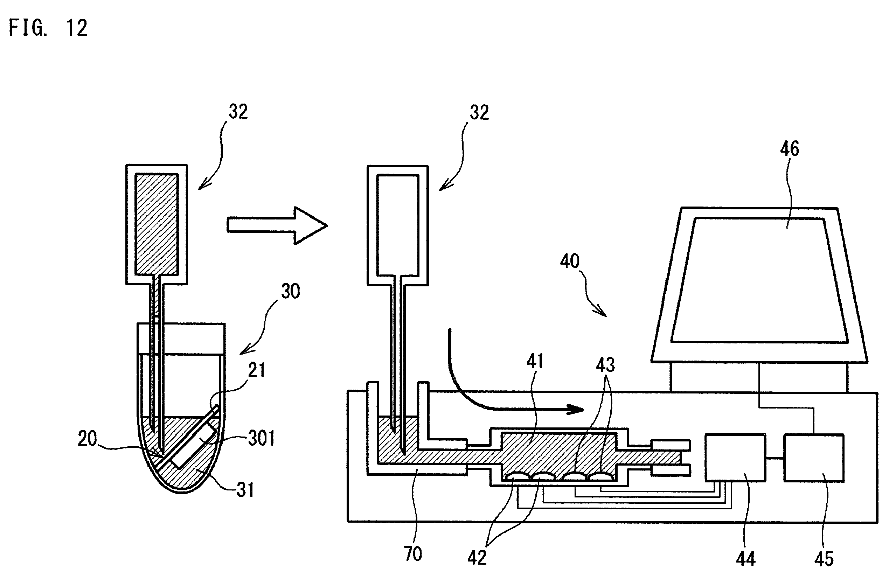

In the AUC the measurement apparatus 100 shown in FIG. 4 to FIG. 11, the gel 301 in which tissue fluid extracted from inside the body is accumulated is set in the set portion 5 of the measurement apparatus 100, and the glucose concentration or the like in the gel 301 is measured. However, an analyte in the gel 301 is collected into pure water in a dedicated container, and the concentration of the collected analyte in the solution may be measured.
For example, as shown in FIG. 12, a gel reservoir 20 provided with the gel 301 with which extraction of an analyte from the skin has ended is immersed in a collection liquid 31 being pure water in a collection tube 30, whereby the analyte accumulated in the gel 301 is collected. After the collection of the analyte has ended, the collection liquid 31 in the collection tube 30 is transferred by a syringe 32 to a measurement portion 41 of a measurement apparatus 40. Similarly to the measurement apparatus 100 described above, the measurement portion 41 is provided with a glucose concentration measurement electrode 42 and a sodium ion concentration measurement electrode 43 which form a detection portion. Then, the glucose concentration and the sodium ion concentration are measured by an electric controller 44 and an analysis portion 45 by use of the above-described method using formulae (2) to (7), whereby the blood glucose AUC is analyzed. The obtained result is outputted on a display portion 46.
As the collection liquid 31, instead of pure water, a liquid that contains a hypertonic aqueous solution having a higher osmotic pressure can be used. The hypertonic aqueous solution can contain at least one auxiliary component different from the measurement target component, for example, at least one member selected from the group consisting of potassium chloride, glycine, and urea. The concentration of the auxiliary component can be set to be not less than 0.2 mmol/L.

[Examples]

Next, Examples using the concentration state analysis method according to the above embodiment will be described. Availability of the above embodiment was examined through these Examples. In particular, Examples 1 to 5 are examples regarding the first analysis process described above, and Example 6 is an example regarding the second analysis process described above. In Example 1, examined was correlation between the AUC value measured from tissue fluid extracted by the minimally invasive tissue fluid extraction technique, and the AUC value calculated from the concentration of the measurement target component obtained through continuous glucose monitoring (hereinafter referred to as CGM value). In Examples 2 to 4, examined was relationship between the AUC value calculated from the CGM values and the ratio of the time period during which the concentration of the measurement target component exceeded a predetermined concentration. In Example 5, examined was relationship between the AUC value measured from tissue fluid extracted by the minimally invasive tissue fluid extraction technique and the ratio of the time period during which the concentration of the measurement target component exceeded a predetermined concentration. In Example 6, it was examined whether the concentration of the measurement target component exceeded a predetermined reference value could be determined by comparing the AUC value with a predetermined threshold value.

<Example 1 > (Comparison between the AUC value measured from tissue fluid extracted by the minimally invasive tissue fluid extraction technique and the AUC value using the CGM values)

In Example 1, the AUC value measured from tissue fluid extracted by the minimally invasive tissue fluid extraction technique, which is for extracting tissue fluid through fine pores formed in the skin of a subject as in the AUC measurement apparatus described in the above embodiment, is compared with the AUC value calculated from the CGM values obtained from the same subject, whereby correlation therebetween is examined.
In Example 1, with respect to 34 subjects, the AUC value in 8 hours of each of a day time period (9 o'clock to 17 o'clock, 8 subjects) and a night time period (22 o'clock to 6 o'clock, 26 subjects) was measured from tissue fluid extracted by the minimally invasive tissue fluid extraction technique. The AUC value measured using the tissue fluid was compared with the AUC value calculated from the CGM measurement values.

The operations from the tissue fluid extraction to the AUC value measurement were performed in the following procedure.

(Pretreatment)

The skin of each of the plurality of subjects was cleaned with alcohol or the like, to remove substances (sweat, dust, etc.) which could be disturbance factors for the result of the measurement, and then, pretreatment was performed. Specifically, fine pores were formed by means of the puncture tool shown in FIG. 8.

(Extraction to accumulation)

A collection member containing a gel was applied to the site where the fine pores were formed, and tissue fluid was extracted. Also to a site where fine pores were not formed, the collection member containing the gel was applied. Specifically, the collection member was applied to each of two sites where fine pores were formed, and each of one site or two sites where fine pores were not formed.

(Collection to measurement)

After 8 hours after the gel of the collection member was attached, each collection member was collected, and only the gel was released from the collection member. The gel attached to the site having fine pores formed therein was immersed in 5000 $\mu$L or 1600 $\mu$L of pure water, and the gel attached to the site having no fine pores formed therein was immersed in 1000 $\mu$L of pure water. The pure water with the gel immersed therein was shaken for 3 hours under an environment of 50°C, whereby components derived from the tissue fluid contained in the gel were collected in the pure water. Then, the liquid was kept under the condition of 4°C until the measurement was performed. By using this collection liquid, the glucose concentration and the sodium ion concentration (Na) were measured in the following manner.

(Measurement of glucose concentration)

100 $\mu$L of the collection liquid from the site having fine pores formed therein, and 250 $\mu$L of the collection liquid from the site having no fine pores formed therein were each used to measure the glucose concentration without being diluted. For the measurement, a glucose oxidase measuring method was used.

(Measurement of sodium ion concentration)

For measurement of the sodium ion concentration, an ion chromatograph manufactured by Dionex or an ion selective electrode manufactured by HORIBA, Ltd. was used. In the measurement using the ion chromatograph, with respect to each of the site having fine pores formed therein and the site having no fine pores formed therein, the collection liquid was diluted 5 fold with pure water, and 100 $\mu$L of the diluted collection liquid was used to measure the sodium ion concentration. In the measurement using the ion selective electrode, the collection liquid from the site having fine pores formed therein was diluted 2 fold with a 8mM potassium chloride solution (manufactured by KANTO KAGAKU), and 250 $\mu$L of the diluted collection liquid was used to measure the sodium ion concentration. The collection liquid from the site having no fine pores formed therein was not diluted and, and 430 $\mu$L thereof was used to measure the sodium ion concentration.

(Calculation of extracted glucose amount)

The extracted glucose amount M (glc) was calculated from the measured glucose concentration, the amount of pure water in which the gel had been immersed, the dilution rate, and the volume of the gel.

(Calculation of electrolyte extraction speed)

The sodium ion concentration C (Na) in the gel was calculated by use of the measured sodium ion concentration, the amount of pure water in which the gel had been immersed, and the dilution rate. Further, the electrolyte extraction speed J at the extraction site of tissue fluid was calculated according to the formula below, from the sodium ion concentration C (Na), the volume V of the gel, the extraction time period t of the tissue fluid.

$$J = C\,(Na) \times V \times 1/t$$

The electrolyte extraction speed J was calculated for each of the tissue fluids extracted from the site having fine pores formed therein and the site having no fine pores formed therein. Then, the value J from the site having no fine pores formed therein was subtracted from the value J from the site having fine pores formed therein, and the obtained value was defined as J (Na). Since the value J obtained from the site having no fine pores formed therein is considered as being derived from sweat, the subtraction was performed in order to use this value as background.

(Measurement of CGM value)

In parallel to the extraction of tissue fluid as described above, the CGM value (blood glucose level) being the glucose concentration in the interstitial fluid subcutaneously collected from the patient was measured. The CGM value was measured every 5 minutes in 8 hours which was the same as the extraction time period of the tissue fluid. For measurement of the CGM value, "Medtronic MiniMed CGM S-Gold" manufactured by Medtronic Japan Co., Ltd. was used.

(Calculation of glucose permeability)

The AUC value was calculated from the measured CGM values. The glucose amount M (glc) obtained above was divided by the AUC value calculated from the CGM values, whereby the glucose permeability P' (glc) was calculated. Further, an approximate straight line of the electrolyte extraction speed J (Na) and the glucose permeability P' (glc) that have been calculated above was obtained, and the glucose permeability P (glc) corresponding to each electrolyte extraction speed J (Na) was obtained by use of the approximate straight line.

(Calculation of predicted AUC value)

A predicted AUC value was obtained by dividing the obtained glucose amount M (glc) by the glucose permeability P (glc).

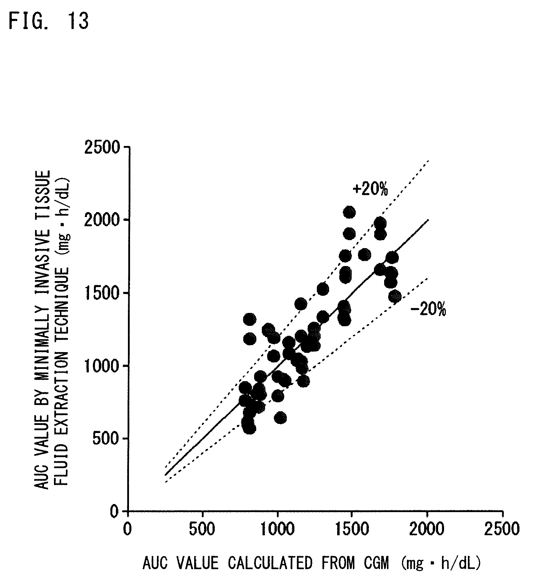

When the predicted AUC value measured using the tissue fluid extracted by the minimally invasive tissue fluid extraction technique and the AUC value calculated from CGM were plotted, the distribution shown in FIG. 13 was obtained. The correlation coefficient between the two kinds of AUC values was $r = 0.86$, and it was found that there is good correlation therebetween.

In Example 1 described above, it was confirmed that there is correlation between the AUC value measured using the tissue fluid extracted by the minimally invasive tissue fluid extraction technique and the AUC value calculated from the CGM values. Therefore, in Examples 2 to 4 described below, by use of the AUC value obtained from the CGM values, relationship between the AUC value and the ratio of the time period during which the concentration of the measurement target component exceeded a predetermined concentration was examined.

<Example 2> (Estimation of the time period during which the CGM value has exceeded 200 mg/dL)

In Example 2, a prediction formula is established by use of the AUC value, and it is examined whether the time period during which the CGM value has exceeded 200 mg/dL can be estimated by the prediction formula. In Example 2, for calculation of the AUC value, the CGM values obtained through the continuous glucose monitoring were used. For the CGM value, interstitial fluid from the subcutaneous tissue of each subject was collected with a "Medtronic MiniMed CGM S-Gold" manufactured by Medtronic Japan Co., Ltd., and the CGM value was obtained every 5 minutes. Then, the obtained CGM values were divided into a data set of CGM values for establishing a prediction formula and a data set of CGM values for evaluating the established prediction formula. Then, with respect to the CGM values of each data set, the AUC value in 8 hours of each of a day time period (9 o'clock to 17 o'clock) and a night time period (22 o'clock to 6 o'clock) was calculated. Further, the ratio of the time period during which the CGM value exceeded 200 mg/dL relative to the AUC value measurement time period (8 hours) was calculated.

The number of data in each data set is as follows.

(1) Data set for prediction formula establishment
day time period: 46 cases; night time period: 66 cases; 112 cases in total
(2) Data set for prediction formula evaluation
day time period: 33 cases; night time period: 50 cases; 83 cases in total

By use of the data set for prediction formula establishment, from the AUC value in 8 hours, a prediction formula for estimating the ratio of the time period during which the CGM value exceeded 200 mg/dL was obtained. In obtaining the prediction formula, the least-squares method was applied to the AUC value of the data set for prediction formula establishment, whereby the constants a, b, and c of the logistic curve (sigmoid curve) indicated by the above-described formula (1) were set. In the prediction formula (1) also shown below, x is the AUC value in 8 hours, and y is the ratio of the time period during which the CGM value has exceeded 200 mg/dL.

[Formula 3]

$$y = \frac{a}{1 + b \times \exp(-cx)} \qquad \cdots (1)$$

In prediction formula (1), the value of each constant a, b, c was set as below.

a=1
b = 5840.12
c = 0.0053

Table 1 below shows, with respect to each of the data set for prediction formula establishment and the data set for prediction formula evaluation, the difference between the estimated value, obtained by putting the AUC value into the prediction formula, of the ratio of the time period during which the CGM value exceeded 200 mg/dL, and the measured value, obtained from the CGM values, of the ratio of the time period during which the CGM value exceeded 200 mg/dL.

[Table 1]

| | Average of difference between estimated value by prediction formula and measured value | Maximum difference between estimated value by prediction formula and measured value |
|---|---|---|
| Data for prediction formula establishment | 0.067 | 0.25 |
| Data for prediction formula evaluation | 0.056 | 0.27 |

With reference to table 1, for each of the data used in prediction formula establishment and the data for prediction formula evaluation, the average of difference between the estimated value obtained from the prediction formula and the measured value obtained from the CGM values is not greater than 0.07, and also, the maximum difference therebetween is not greater than 0.27. Thus, it was found that estimated values that are close to the measured values can be obtained by use of the prediction formula.

Furthermore, when measured values of each data set were plotted together with the curve indicated by the set prediction formula, the distribution as in FIG. 14 was obtained. It was found that the curve indicated by the prediction formula is approximate to either set of measured values.

<Example 3> (Estimation of the time period during which the CGM value has exceeded 180 mg/dL)

In Example 2 described above, the reference value for the CGM value was set at 200 mg/dL, whereas in Example 3, the reference value for the CGM value was set at 180 mg/dL, and the similar examination was performed by use of the same CGM data sets as in Example 2.

By use of the data set for prediction formula establishment, from the AUC value in 8 hours, a prediction formula for estimating the ratio of the time period during which the CGM value exceeded 180 mg/dL was obtained. In obtaining the prediction formula, the least-squares method was applied to the data set for prediction formula establishment, whereby the constants a, b, and c of the logistic curve (sigmoid curve) indicated by formula (1) described above were set. Specifically, the value of each constant a, b, c was set as below.

a=1
b = 18421

c = 0.0067

Table 2 below shows, with respect to each of the data set for prediction formula establishment and the data set for prediction formula evaluation, the difference between the estimated value, obtained by putting the AUC value into the prediction formula, of the ratio of the time period during which the CGM value exceeded 180 mg/dL, and the measured value, obtained from the CGM values, of the ratio of the time period during which the CGM value exceeded 180 mg/dL.

[Table 2]

| | Average of difference between estimated value by prediction formula and measured value | Maximum difference between estimated value by prediction formula and measured value |
|---|---|---|
| Data for prediction formula establishment | 0.069 | 0.28 |
| Data for prediction formula evaluation | 0.064 | 0.27 |

With reference to table 2, for each of the data used in prediction formula establishment and the data for prediction formula evaluation, the average of difference between the estimated value obtained from the prediction formula and the measured value obtained from the CGM values is not greater than 0.07, and also, the maximum difference therebetween is not greater than 0.28. Thus, it was found that estimated values that are close to the measured values can be obtained by use of the prediction formula.

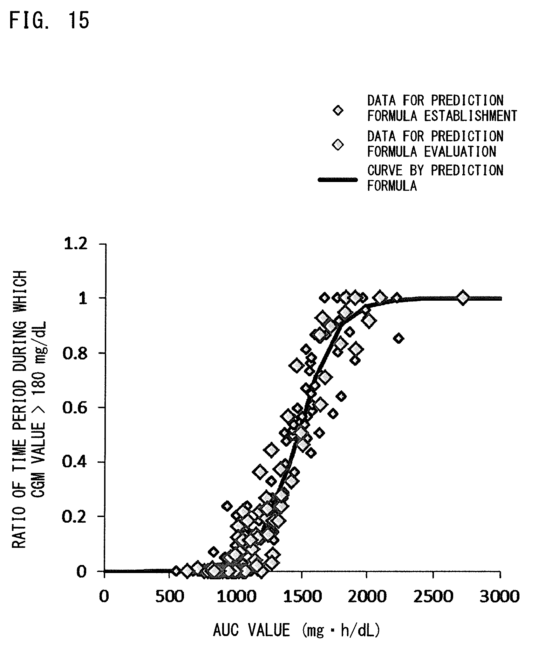

When the measured values of each data set were plotted together with the curve indicated by the set prediction formula, the distribution as in FIG. 15 was obtained. It was found that the curve indicated by the prediction formula was approximate to either set of measured values.

<Example 4> (Estimation of the ratio of the time period during which the CGM value has exceeded 180 mg/dL using the AUC value calculated from the CGM values)

In Example 4, with respect to the AUC value calculated from the CGM values, the ratio of the time period during which the CGM value has exceeded 180 mg/dL relative to the measurement time period of the AUC value is estimated by prediction formula (1) obtained in Example 3. Then, this estimated ratio is compared with the ratio of the time period during which the CGM value actually exceeded 180 mg/dL.

In Example 4, the same data as used in Example 2 was used, and the ratio of the time period during which the CGM value exceeded 180 mg/dL was estimated according to the prediction formula established in Example 3 by use of the AUC value calculated from the CGM values. Then, this estimated value was compared with the ratio of the time period during which the CGM value actually exceeded 180 mg/dL.

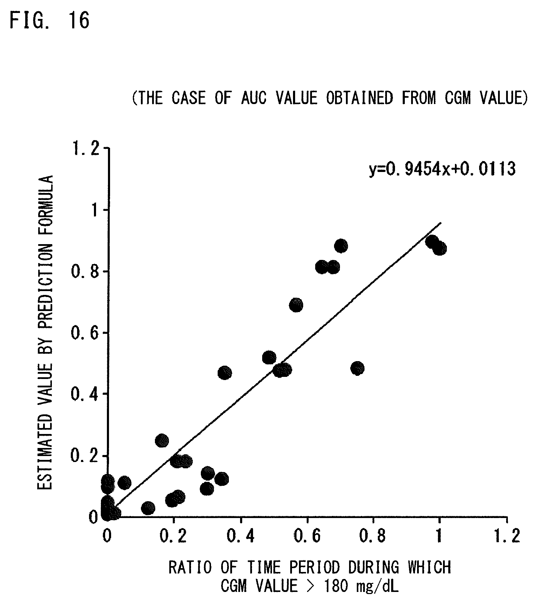

FIG. 16 shows relationship, by plotting, between the estimated value obtained by putting the AUC value obtained from the CGM value into the prediction formula, and the ratio of the time period during which the CGM value actually exceeded 180 mg/dL. With reference to the result shown in FIG. 16, the correlation coefficient between the estimated value obtained from the AUC value and the ratio of the time period during which the CGM value actually exceeded 180 mg/dL was r = 0.93, and it was found that there is good correlation therebetween.

<Example 5> (Estimation of the ratio of the time period during which the CGM value has exceeded 180 mg/dL using the AUC value measured using the tissue fluid extracted by the minimally invasive tissue fluid extraction technique)

In Example 5, with respect to the AUC value measured using the tissue fluid extracted by the minimally invasive tissue fluid extraction technique as with the AUC measurement apparatus described in each embodiment, the ratio of the time period during which the CGM value has exceeded 180 mg/dL relative to the measurement time period of the AUC value is estimated by the prediction formula (1) obtained in Example 3. Then, this estimated ratio is compared with the ratio of the time period during which the CGM value actually exceeded 180 mg/dL.

In Example 5, the same data as used in Example 1 was used, and the time period during which the CGM value exceeded 180 mg/dL was estimated according to the prediction formula established in Example 3 by use of the AUC value measured using the tissue fluid extracted by the minimally invasive tissue fluid extraction technique. Then, this estimated value was compared with the ratio of the time period during which the CGM value actually exceeded 180 mg/dL.

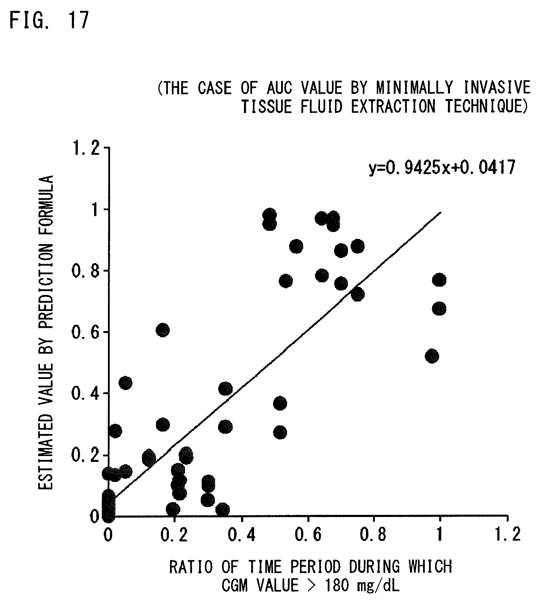

FIG. 17 shows relationship, by plotting, between the estimated value obtained by putting the AUC value measured using the tissue fluid extracted by the minimally invasive tissue fluid extraction technique into the prediction formula, and the ratio of the time period during which the CGM value actually exceeded 180 mg/dL. With reference to the result shown in FIG. 17, the correlation coefficient between the estimated value obtained from the AUC value by the minimally invasive tissue fluid extraction technique and the ratio of the time period during which the CGM value exceeded 180 mg/dL was r=0.83, and it was found that there is good correlation therebetween.

<Example 6> (Extraction of subjects whose CGM value exceeded 200 mg/dL)

In Example 6, it is examined whether the blood glucose level being the concentration of the measurement target component has exceeded a predetermined reference value, i.e., whether the blood glucose level is in a hyperglycemic state, can be determined by comparing the AUC value with a predetermined threshold value. In Example 1 described above, it was confirmed that there is correlation between the AUC value measured by using the tissue fluid extracted by the minimally invasive tissue fluid extraction technique and the AUC value calculated from the CGM values. Therefore, also in Example 6, the same CGM values as those used in Example 2 were used. Using these CGM values, a data set of CGM values for setting a cutoff which is a predetermined threshold value, and a data set of CGM values for evaluating the set cutoff were prepared. Then, with respect to each set of CGM values, the AUC value in 8 hours of each of a day time period (9 o'clock to 17 o'clock) and a night time period (22 o'clock to 6 o'clock) was calculated.
The number of data of each data set is as follows.

(1) Data set for cutoff setting
day time period: 46 cases; night time period: 66 cases; 112 cases in total
(2) Data set for evaluation
day time period: 33 cases; night time period: 50 cases; 83 cases in total

The predetermined reference value for the blood glucose level was set at 200 mg/dL serving as an index for hyperglycemic state. The cutoff of the AUC value was set by performing ROC analysis by using the data set for cutoff. Specifically, the cutoff was set at 1177.25 (mg·h/dl). Using this cutoff, with respect to the data set for evaluation, it was examined whether the CGM value exceeded 200 mg/dL, i.e., whether the blood glucose level was in a hyperglycemic state could be determined. Table 3 below shows the result.

[Table 3]

| | |
|---|---|
| Sensitivity | 0.775 |
| Specificity | 0.907 |
| PPV | 0.886 |
| NPV | 0.813 |

Table 3 shows the determination capability regarding the presence/absence of CGM value that exceeds 200 mg/dL, through comparison between the set cutoff and the AUC value. In table 3, "sensitivity" is the ratio of cases that include CGM values exceeding 200 mg/dL and whose AUC values exceed the cutoff, relative to the cases that include CGM values exceeding 200 mg/dL. "Specificity" is the ratio of cases that include CGM values being not greater than 200 mg/dL and whose AUC values are not greater than the cutoff, relative to the cases that include CGM values being not greater than 200 mg/dL. "PPV" is the positive predictive value, and is the ratio of the cases whose AUC values exceed the cutoff and whose CGM values exceed 200 mg/dL, relative to the cases whose AUC values exceed the cutoff. "NPV" is the negative predictive value, and is the ratio of the cases whose AUC values are not greater than the cutoff and whose CGM values in the measurement time period are all smaller than or equal to 200 mg/dL, relative to the cases whose AUC values are not greater than the cutoff.
With reference to table 3, for each of sensitivity, specificity, PPV, and NPV, a value about 0.8 or higher could be obtained. Therefore, it was found that, by comparing the AUC value with a predetermined threshold value, the presence/absence of a high concentration state of the measurement target component can be determined.

**Claims**

**1.** A method for analyzing a concentration state of a measurement target component in blood, the method comprising:

obtaining a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid;

obtaining a value of an area under a blood concentration time curve of the measurement target component on the basis of the value regarding the amount of the measurement target component; and

calculating a value regarding a ratio of a time period during which a concentration of the measurement target component has exceeded a predetermined concentration relative to the extraction time period, on the basis of the value of the area under the blood concentration time curve.

2. The method for analyzing the concentration state of the measurement target component in blood of claim 1, further comprising:

obtaining a value regarding an amount of an electrolyte in the collection member, wherein
the value of the area under the blood concentration time curve is obtained on the basis of the value regarding the amount of the measurement target component and the value regarding the amount of the electrolyte.

3. The method for analyzing the concentration state of the measurement target component in blood of claim 2, wherein the value of the area under the blood concentration time curve is obtained on the basis of the value regarding the amount of the measurement target component, the value regarding the amount of the electrolyte, and the extraction time period.

4. The method for analyzing the concentration state of the measurement target component in blood of any one of claims 1 to 3, wherein
the collection member includes a gel in which to accumulate the tissue fluid extracted for the extraction time period.

5. The method for analyzing the concentration state of the measurement target component in blood of any one of claims 1 to 4, wherein
the process for accelerating the extraction of the tissue fluid is a process for forming fine pores in skin of the organism, and
the collection member accumulates the tissue fluid extracted through the fine pores.

6. The method for analyzing the concentration state of the measurement target component in blood of any one of claims 1 to 5, wherein
the value regarding the ratio of the time period during which the concentration of the measurement target component has exceeded the predetermined concentration relative to the extraction time period is calculated by applying the value of the area under the blood concentration time curve to a formula below:

[Formula]

$$y = \frac{a}{1 + b \times \exp(-cx)}$$

(where y is the value regarding the ratio of the time period during which the concentration of the measurement target component has exceeded the predetermined concentration, x is the value of the area under the blood concentration time curve, and a, b, and c are each a constant).

7. The method for analyzing the concentration state of the measurement target component in blood of any one of claims 1 to 6, wherein
the measurement target component is glucose.

8. The method for analyzing the concentration state of the measurement target component in blood of claim 7, wherein the predetermined concentration is a value in a range of not less than 180 mg/dL and not greater than 200 mg/dL.

9. A method for analyzing a concentration state of a measurement target component in blood, the method comprising:

obtaining a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid;

obtaining a value of an area under a blood concentration time curve of the measurement target component on the basis of the value regarding the amount of the measurement target component;

comparing the value of the area under the blood concentration time curve with a predetermined threshold value; and

determining, on the basis of a result of the comparison, whether a concentration of the measurement target component has exceeded a predetermined concentration.

10. An apparatus configured to analyze a concentration state of a measurement target component in blood, the apparatus comprising:

a detection portion configured to obtain a value regarding an amount of the measurement target component in a collection member in which tissue fluid has been accumulated in advance, the tissue fluid having been extracted for a predetermined extraction time period from an organism which had been subjected to a process for accelerating the extraction of the tissue fluid;

an analysis portion configured to obtain a value of an area under a blood concentration time curve of the measurement target component on the basis of the value regarding the amount of the measurement target component obtained by the detection portion; and

a calculation portion configured to calculate a value regarding a ratio of a time period during which a concentration of the measurement target component has exceeded a predetermined concentration relative to the extraction time period of the measurement target component, on the basis of the value of the area under the blood concentration time curve obtained by the analysis portion.

11. The apparatus of claim 10, further comprising
a set portion in which to set the collection member.

12. The apparatus of claim 10 or 11, wherein
the detection portion obtains a value regarding an amount of an electrolyte in the collection member, and
the analysis portion obtains the value of the area under the blood concentration time curve on the basis of the value regarding the amount of the measurement target component and the value regarding the amount of the electrolyte.

13. The apparatus of claim 12, wherein
the analysis portion obtains the value of the area under the blood concentration time curve on the basis of the value regarding the amount of the measurement target component, the value regarding the amount of the electrolyte, and the extraction time period.

14. The apparatus of any one of claims 10 to 13, wherein
the collection member includes a gel in which to accumulate the tissue fluid extracted for the extraction time period.

15. The apparatus of any one of claims 10 to 14, wherein
the process for accelerating the extraction of the tissue fluid is a process for forming fine pores in skin of the organism, and
the collection member accumulates the tissue fluid extracted through the fine pores.

FIG. 1

CONCENTRATION STATE
ANALYZER                           700

(AUC VALUE)

INPUT PORTION                      706

CONTROLLER                         701

CPU                                703

STORAGE PORTION                    704

100

AUC
MEASUREMENT                        COMMUNICATION
APPARATUS                          INTERFACE        705

DISPLAY PORTION                    702

FIG. 2

FIG. 3

START

S1
OBTAIN AUC VALUE

S2
(FIRST ANALYSIS PROCESS)   SELECT ANALYSIS PROCESS   (SECOND ANALYSIS PROCESS)

S3
PUT AUC VALUE INTO PREDICTION FORMULA AND CALCULATE FIRST ANALYSIS VALUE

S4
COMPARE AUC VALUE WITH THRESHOLD VALUE

S5
DETERMINE CONCENTRATION OF MEASUREMENT TARGET COMPONENT (OBTAIN SECOND ANALYSIS VALUE)

S6
OUTPUT PROCESS RESULT

END

FIG. 4

FIG. 5

FIG. 6

FIG. 7

FIG. 8

FIG. 9

FIG. 10

FIG. 11

FIG. 12

FIG. 13

FIG. 14

FIG. 15

Legend:
- ◇ DATA FOR PREDICTION FORMULA ESTABLISHMENT
- ◇ DATA FOR PREDICTION FORMULA EVALUATION
- —— CURVE BY PREDICTION FORMULA

Y-axis: RATIO OF TIME PERIOD DURING WHICH CGM VALUE > 180 mg/dL (1.2, 1, 0.8, 0.6, 0.4, 0.2, 0)

X-axis: AUC VALUE (mg·h/dL) (0, 500, 1000, 1500, 2000, 2500, 3000)

FIG. 16

(THE CASE OF AUC VALUE OBTAINED FROM CGM VALUE)

FIG. 17

(THE CASE OF AUC VALUE BY MINIMALLY INVASIVE
TISSUE FLUID EXTRACTION TECHNIQUE)

$y=0.9425x+0.0417$

ESTIMATED VALUE BY PREDICTION FORMULA

RATIO OF TIME PERIOD DURING WHICH
CGM VALUE > 180 mg/dL

Europäisches
Patentamt

European
Patent Office

Office européen
des brevets

# EUROPEAN SEARCH REPORT

Application Number

EP 16 18 6294

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 198 774 A1 (SYSMEX CORP [JP]) 23 June 2010 (2010-06-23) * paragraph [0011] - paragraph [0035] * * paragraph [0076] * * figures 1,2,6-12,30,31 * | 1-15 | INV. A61B5/145 A61B5/1486 |
| A | EP 2 368 497 A1 (SYSMEX CORP [JP]) 28 September 2011 (2011-09-28) * paragraph [0011] - paragraph [0012] * * paragraph [0016] - paragraph [0017] * * paragraph [0022] - paragraph [0023] * * paragraph [0029] * * paragraph [0033] - paragraph [0034] * * paragraph [0040] - paragraph [0057] * * paragraph [0062] * * figures 1,2,4-12,15A,15B * | 1-15 | |
| A | US 2013/035871 A1 (MAYOU PHIL [US] ET AL) 7 February 2013 (2013-02-07) * paragraph [0002] * * paragraph [0009] * * paragraph [0072] * * figures 1,5A-5E * | 1-15 | TECHNICAL FIELDS SEARCHED (IPC) A61B |
| A | US 2003/211617 A1 (JONES STEVEN PAUL [US]) 13 November 2003 (2003-11-13) * paragraph [0001] * * paragraph [0018] * * figures 1,3 * | 1-15 | |
| A | WO 2008/111935 A1 (BAYER HEALTHCARE LLC [US]; POWER BARRY D [US]; CULVER JEFFREY A [US]) 18 September 2008 (2008-09-18) * paragraph [0001] * * paragraph [0005] - paragraph [0007] * * paragraph [0013] - paragraph [0014] * * figures 1,2 * | 1-15 | |

The present search report has been drawn up for all claims

| Place of search | Date of completion of the search | Examiner |
|---|---|---|
| Berlin | 17 January 2017 | Völlinger, Martin |

EPO FORM 1503 03.82 (P04C01)

## ANNEX TO THE EUROPEAN SEARCH REPORT
## ON EUROPEAN PATENT APPLICATION NO.

EP 16 18 6294

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2017

| Patent document cited in search report | | Publication date | Patent family member(s) | | Publication date |
|---|---|---|---|---|---|
| EP 2198774 | A1 | 23-06-2010 | CN 101762629 A | | 30-06-2010 |
| | | | EP 2198774 A1 | | 23-06-2010 |
| | | | JP 5470010 B2 | | 16-04-2014 |
| | | | JP 2010169662 A | | 05-08-2010 |
| | | | US 2010160758 A1 | | 24-06-2010 |
| EP 2368497 | A1 | 28-09-2011 | EP 2368497 A1 | | 28-09-2011 |
| | | | JP 5419771 B2 | | 19-02-2014 |
| | | | JP 2011200565 A | | 13-10-2011 |
| | | | US 2011237919 A1 | | 29-09-2011 |
| US 2013035871 | A1 | 07-02-2013 | US 2013035575 A1 | | 07-02-2013 |
| | | | US 2013035865 A1 | | 07-02-2013 |
| | | | US 2013035871 A1 | | 07-02-2013 |
| | | | WO 2013022775 A1 | | 14-02-2013 |
| US 2003211617 | A1 | 13-11-2003 | NONE | | |
| WO 2008111935 | A1 | 18-09-2008 | DK 2134251 T3 | | 24-11-2014 |
| | | | EP 2134251 A1 | | 23-12-2009 |
| | | | US 2010041083 A1 | | 18-02-2010 |
| | | | WO 2008111935 A1 | | 18-09-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010013808 A